## Europäisches Patentamt

## European Patent Office

## Office européen des brevets

(19)

(11) Veröffentlichungsnummer: **0 068 438**
**B1**

(12) **EUROPÄISCHE PATENTSCHRIFT**

(45) Veröffentlichungstag der Patentschrift:
29.05.85

(51) Int. Cl.⁴: **C 12 Q 1/28**

(21) Anmeldenummer: **82105538.1**

(22) Anmeldetag: **24.06.82**

(54) **Mittel und Verfahren zum Nachweis von Wasserstoffperoxid.**

(30) Priorität: **30.06.81 DE 3125667**

(43) Veröffentlichungstag der Anmeldung:
**05.01.83 Patentblatt 83/1**

(45) Bekanntmachung des Hinweises auf die Patenterteilung:
**29.05.85 Patentblatt 85/22**

(84) Benannte Vertragsstaaten:
**AT BE CH DE FR GB IT LI LU NL SE**

(56) Entgegenhaltungen:
**EP - A - 0 024 578**
**DE - A - 2 833 741**
**DE - A - 3 003 490**

**CLINICAL CHEMISTRY, Band 27, Nr. 3, März 1981, Seiten 375-379, Easton, Pennsylvania, USA F. GRILLO et al.: "Improved method for determination of high-density-lipoprotein cholesterol II. Enzymic determination of cholesterol in high-density lipoprotein fractions with a sensitive reagent"**

(73) Patentinhaber: **Boehringer Mannheim GmbH, Sandhoferstrasse 116, D-6800 Mannheim 31 (DE)**

(72) Erfinder: **Pakebusch, Bernd, Bergstrasse 20, D-6711 Hessheim (DE)**
Erfinder: **Carstensen, Carsten, Folradstrasse 8, D-6711 Heuchelheim (DE)**
Erfinder: **Sojka, Bernward, Dr.rer.nat., Bunsenstrasse 4, D-6806 Viernheim (DE)**
Erfinder: **Lange, Hans-Rudolf, Danzigerstrasse 3, D-6840 Lampertheim (DE)**

## Beschreibung

Die Erfindung betrifft Mittel und Verfahren zum Nachweis von Wasserstoffperoxid durch oxidative Kondensation von Chromogenen in Gegenwart von Peroxidase.

Die analytische Bestimmung von Wasserstoffperoxid hat für die medizinische Diagnostik eine erhebliche Bedeutung, da bei einer großen Anzahl von wichtigen Nachweisverfahren Wasserstoffperoxid als Zwischenprodukt gebildet wird, welches danach durch Umsetzung mit geeigneten chromogenen Substanzen, überwiegend in Gegenwart einer Peroxidase (POD) als Katalysator, in eine optisch erfaßbare Substanz überführt wird. Das Ausmaß der Bildung des optisch erfaßbaren Produktes dient als Maß für die Menge des Wasserstoffperoxids beziehungsweise als Maß für die Menge des Wasserstoffperoxid bildenden Substrates. Beispielhaft für solche Reaktionen seien der Nachweis von Glucose/Glucoseoxidase, (GOD), Cholesterin/Cholesterinoxidase (CHO)/Cholesterinesterase (CHE) und Harnsäure/Uricase genannt. Für diese Reaktion sind zahlreiche Chromogene beziehungsweise Indikatorsysteme vorgeschlagen worden. Eines der am häufigsten angewandten Indikatorsysteme ist das von Trinder (Biochem. 6 (1969) 24—27), bei dem Phenol mit 4-Aminoantipyrin (4-AAP) in Gegenwart von POD unter Einwirkung von $H_2O_2$ oxidativ zu einem Farbstoff gekuppelt wird. Anstelle des Phenols können auch Phenolderivate, Anilinderivate, Naphthol, Naphtholderivate oder ähnlich reagierende Substanzen eingesetzt werden. Anstelle des 4-Aminoantipyrins können auch andere Aminoantipyrinderivate, Vanillindiaminsulfonsäure, Methylbenzolthiazolonhydrazon (MBTH), sulfoniertes Methylbenzothiazolonhydrazon (SMBTH) und ähnlich reagierende Verbindungen eingesetzt werden.

Obwohl 4-AAP in dieser Reaktion als empfindliches Reagenz bekannt ist, hat es doch gewisse Nachteile. Insbesondere zeigt sich bei den handelsüblichen Reagenzien, daß bei einer Auftragung der Konzentration des gebildeten Farbstoffs gegen die Konzentration des oxidierenden Agens ($H_2O_2$ bzw. $H_2O_2$ bildendes Substrat) zwar in weiten Konzentrationsbereichen eine lineare Abhängigkeit besteht, jedoch für geringe Konzentrationen negative Farbstoffkonzentrationen extrapoliert werden. Dies ist darauf zurückzuführen, daß auch hochgereinigtes 4-AAP in der Testmischung bei Belastung oder längerer Lagerung in geringem Maße Zersetzungsprodukte unbekannter Konstitution bildet, die $H_2O_2$ reduzieren und damit der eigentlichen Nachweisreaktion entziehen.

Es stellte sich deshalb die Aufgabe, 4-AAP so zu stabilisieren, daß sich in den Testsystemen auch bei längerer Lagerung keine oder nur noch vernachlässigbare Mengen der vorstehenden Zersetzungsprodukte bilden.

Überraschenderweise wurde gefunden, daß sich diese Aufgabe dadurch lösen läßt, daß man das 4-AAP mit einer geringen Menge eines alkalischen Puffers im pH-Bereich von 8,5—14,0 vermischt lagert und erst kurz vor der Testreaktion durch Zugabe einer größeren Puffermenge vom pH-Wert 5,0—8,0 in einen für den Ablauf enzymatischen Reaktion notwendigen Bereich bringt.

Die beiden Zusammensetzungen werden dabei in einem für den Nachweis geeigneten Mengenverhältnis zueinander in einer Verpackungseinheit oder einer Gebrauchseinheit zusammengefaßt. Unter Verpackungseinheit wird dabei verstanden, daß sich die in zwei verschiedenen Behältnissen, meistens in einer für mehrere Teste gedachten Menge, abgepackten Zubereitungen in einer gemeinsamen Umverpackung befinden.

Unter Gebrauchseinheit wird verstanden, daß die Zubereitungen sich in einer gemeinsamen Verpackung befinden, aber physikalisch voneinander getrennt sind und erst durch die Zugabe der Testflüssigkeit vereint werden. Beispielhaft sei erwähnt die Separierung in zwei Tabletten, Mehrschichttabletten, Mehrschichtlyophilisate gegebenenfalls in einer als Reaktionsgefäß dienenden Küvette etc. Besonders bevorzugt ist es die beiden Zusammensetzungen auf saugfähige Träger zu imprägnieren und Stücke davon auf einem Handgriff zu befestigen, so daß beim Eintauchen in die Testflüssigkeit die gewünschte Menge beider Komponenten gleichzeitig abgegeben wird.

Da aromatische Amine normalerweise im neutralen oder sauren Milieu relativ stabil sind, aber im alkalischen Milieu einer oxidativen Zersetzung unterliegen, scheint es außerordentlich überraschend, daß 4-AAP gerade in diesem Bereich stabilisiert werden kann.

Als alkalische Puffer können alle diejenigen verwendet werden, die den gewünschten pH-Bereich einstellen und mit den anderen Reagenzien, insbesondere den verwendeten Enzymen, verträglich sind. Beispielhaft seien Alkali-Carbonat/Bicarbonatpuffer, Borat-, Phosphat-, Glycin-, Veronal-, Tris- und Triethanolamin-Puffer genannt.

Als neutrale oder saure Puffer, in denen die Nachweisreaktion durchgeführt wird, seien beispielsweise Phosphat-, Citrat- und Acetat-Puffer genannt. Da die zur Bildung von $H_2O_2$ aus dem Substrat in den oben erwähnten Testen benötigten Enzyme und sonstige Hilfsreagenzien und auch die Peroxidase normalerweise in saurem Milieu stabiler sind als im alkalischen Milieu, ist es vorteilhaft, diese von 4-AAP zu trennen und zusammen mit dem »sauren« Puffer zu lagern. Das fertige Reagenzsystem wird dann kurz vor der Durchführung der Reaktion durch Mischen der beiden Komponenten im geeigneten Verhältnis hergestellt. Die Lagerung erfolgt für beide Komponenten üblicherweise in fester Form als Lyophilisat, Pulver, Tablette oder imprägniert auf saugfähige Träger, aus denen durch Zusatz von Wasser das Reagenz hergestellt wird, oder die auch direkt in die zu untersuchende gelöste Probe eingegeben werden können.

In den folgenden Beispielen sind einige Anwendungen der Erfindung beschrieben.

**0 068 438**

Die in den folgenden Beispielen angegebenen Enzymaktivitäten (U) entsprechen der internationalen Einheit, gemäß der ein U $10^{-6}$ Mol/Substrat/Minute bei 25° C umsetzt. Ein U = 16 · 67 n hat.

Beispiel 1

Glucose-Farbtest mit 4-Aminoantipyrin/GOD, POD

Ein Reagenzstreifen von 10 mm Breite und ca. 98 mm Länge, an welchem am unteren Ende 2 voneinander getrennte Bezirke der Fläche $10 \times 15$ mm befestigt sind und wovon der eine 7,7 mg 4-Aminoantipyrin zusammen mit 757 µg $NaHCO_3$ und 878 µg $Na_2CO_3$, imprägniert auf Filterpapier (598 Schleicher und Schüll) aus wäßriger Lösung (pH = 10,5) und der andere 900 U Glucoseoxidase sowie 55 U Peroxidase imprägniert auf das Polyamid/Cellulosevlies (VS 532, Binzer) aus 0,1molaren Phosphatpuffer pH = 6,0, enthält, wird in 50 ml einer 0,1 molaren Phosphatpufferlösung pH = 7,0, die 10 mMol/1 Phenol enthält, eluiert.

In 2 ml dieser Lösung wird 0,1 ml Uranylacetat enteiweißtes Serum zupipettiert, gut gemischt und das Reaktionsgemisch 30 Minuten bei Raumtemperatur stehen gelassen.

Die nach dieser Zeit abgeschlossene Farbreaktion wird wahlweise bei 505 oder 546 nm gemessen. Die Linearitätsprüfung von 0–600 mg Glucose/l mit 6 beziehungsweise 12 Wochen bei 35° C unter Licht- und Feuchtigkeitsausschluß gelagerten Reagenzstreifen, eluiert in frischer 0,1 molarer Phosphatpuffer-Lösung von pH = 7,0 mit 10 mMol/l Phenol, zeigt, daß praktisch ein vernachlässigbarer negativer Achsenabschnitt auftritt.

| Reagenzstreifen | Regression |
|---|---|
| unbelastet | y = 0,00230 x + 0,0019 |
| 6 Wochen bei 35° C | y = 0,00231 x − 0,0022 |
| 12 Wochen bei 35° C | y = 0,00230 x − 0,0040 |

Vergleicht man in analoger Weise hergestellte Reagenzstreifen, bei dem das 4-Aminoantipyrin-Papier mit Phosphatpuffer auf pH = 7,0 abgepuffert ist, so ergibt sich mit steigender Belastungszeit ein zunehmender negativer Achsenabschnitt.

| Reagenzstreifen | Regression |
|---|---|
| unbelastet | y = 0,00227 x − 0,0026 |
| 6 Wochen bei 35° C | y = 0,00220 x − 0,013 |
| 12 Wochen bei 35° C | y = 0,00230 x − 0,029 |

Beispiel 2

Cholesterin-Farbtest mit 4-Aminoantipyrin (CHE, CHO, POD)

Ein Reagenzstreifen von 6 mm Breite und ca. 75 mm Länge, an welchem am unteren Ende 2 voneinander getrennte Bezirke der Fläche $6 \times 6$ mm befestigt sind und wovon der eine 410 µg 4-Aminoantipyrin zusammen mit 84 µg $NaHCO_3$ sowie 106 µg $Na_2CO_3$ imprägniert auf Filterpapier (598 F Schleicher u. Schüll) aus wäßriger Lösung (pH = 10,0) und der andere 0,3 U Cholesterinoxidase, 0,6 U Cholesterinesterase sowie 4,7 U Peroxidase imprägniert auf Papier 598 F aus 0,1 molaren Phosphatpuffer-Lösung pH 7,5, die 10 mmol/l Phenol enthält, wird eluiert.

Zu dieser Lösung wird 0,02 ml Serum zupipettiert, gut gemischt und das Reaktionsgemisch 30 Minuten bei Raumtemperatur stehen gelassen. Die nach dieser Zeit abgeschlossene Farbreaktion wird wahlweise bei 505 oder 546 nm gemessen. Die Linearitätsprüfung von 0–400 mg Cholesterin/dl mit 6 beziehungsweise 12 Wochen bei 35° C unter Licht- und Feuchtigkeitsausschluß gelagerten Reagenzstreifen eluiert in frischer Phosphatpuffer-Lösung pH = 7,5, die 10 mmol/l Phenol enthält, zeigt, daß kein negativer Achsenabschnitt auftritt.

3

| Reagenzstreifen | Regression |
|---|---|
| unbelastet | $y = 0{,}0012\,x + 0{,}003$ |
| 12 Wochen bei 35° C | $y = 0{,}0012\,x - 0{,}003$ |

Vergleicht man in analoger Weise hergestellte Reagenzstreifen, bei dem das 4-Aminoantipyrin-Papier mit Phosphatpuffer auf pH = 7,0 abgepuffert ist, so ergibt sich mit steigender Belastungszeit ein zunehmender negativer Achsenabschnitt.

| Reagenzstreifen | Regression |
|---|---|
| unbelastet | $y = 0{,}0012\,x + 0{,}0007$ |
| 12 Wochen bei 35° C | $y = 0{,}0012\,x - 0{,}0160$ |

## Beispiel 3

Stabilisierung von 4-Aminoantipyrin mit verschiedenen 0,1 molaren Puffern von pH = 10,0

Linearitätsprüfung von 0—600 mg Glucose/dl bei 546 nm

| Puffer | 0,1 M NaHCO$_3$/Na$_2$CO$_3$ | 0,1 M Piperazin | 0,1 M Glycin |
|---|---|---|---|
| Reagenzstreifen | | Regression | |
| unbelastet | $y = 0{,}00230\,x + 0{,}0019$ | $y = 0{,}00227\,x - 0{,}0032$ | $y = 0{,}00220\,x - 0{,}0022$ |
| 6 Wochen bei 35° C | $y = 0{,}00231\,x - 0{,}0022$ | $y = 0{,}00227\,x - 0{,}0031$ | $y = 0{,}00226\,x - 0{,}0038$ |

| Puffer | 0,1 M Tris | 0,1 M Triäthanolamin | 0,1 M Veronal |
|---|---|---|---|
| Reagenzstreifen | | Regression | |
| unbelastet | $y = 0{,}00226\,x - 0{,}0010$ | $y = 0{,}00215\,x - 0{,}0004$ | $y = 0{,}00219\,x - 0{,}0040$ |
| 6 Wochen bei 35° C | $y = 0{,}00226\,x + 0{,}0044$ | $y = 0{,}00225\,x - 0{,}0044$ | $y = 0{,}00223\,x - 0{,}0034$ |

## Patentansprüche

1. Mittel zum Nachweis von Wasserstoffperoxid beziehungsweise Wasserstoffperoxiod bildenden Substraten, bestehend aus 4-Aminoantipyrin und einem aromatischen Amin oder Phenol und einem neutralen bis schwach sauren Puffer sowie gegebenenfalls anderen für die Trinderreaktion bekannten Zusatzstoffen und Reagenzien, die mit dem Substrat H$_2$O$_2$ bilden, dadurch gekennzeichnet, daß das 4-Aminoantipyrin in vor Gebrauch unvermischbarem Zustand von dem ersten Puffer und gegebenenfalls den übrigen Reagenzien getrennt vorliegt und mit einer geringen Menge eines zweiten Puffers mit einem pH-Wert von 8,5—14,0 vermischt ist, wobei die zwei Zusammensetzungen in einem für den Nachweis geeigneten Mengenverhältnis zueinander in einer Verpackungseinheit oder in einer Gebrauchseinheit zusammengefaßt sind.

2. Verfahren zum Nachweis von Wasserstoffperoxid beziehungsweise Wasserstoffperoxid bildenden Substraten mittels 4-Aminoantipyrin und einem aromatischen Amin oder Phenol und einem neutralen bis schwach sauren Puffer sowie gegebenenfalls anderen für die Trinderreaktion bekannten Zusatzstoffen und Reagenzien die mit dem Substrat H$_2$O$_2$ bilden, dadurch gekennzeichnet, daß man mit einer geringen Menge eines zweiten Puffers mit einem pH-Wert von 8,5—14,0 stabilisiertes 4-Aminoantipyrin kurz vor Gebrauch mit einem Überschuß des ersten Puffers sowie gegebenenfalls den übrigen Reagenzien zusammenfügt, das Substrat hinzufügt und die entstehende Verfärbung visuell oder photometrisch auswertet.

3. Verwendung eines aus zwei räumlich getrennten Zusammensetzungen bestehenden Mittels, von denen die eine 4-Aminoantipyrin und eine geringe Menge eines Puffers mit einem pH-Wert von 8,5—14,0 und die andere einen Überschuß eines neutralen oder sauren Puffers enthält und beide Zusammensetzungen weitere für die Trinderreaktion oder H$_2$O$_2$-Bildung notwendige Reagenzien ent-

halten zum Nachweis von Wasserstoffperoxid beziehungsweise Wasserstoffperoxid bildenden Substraten, wobei die zwei Zusammensetzungen in einem für den Nachweis geeigneten Mengenverhältnis zueinander in einer Verpackungseinheit oder in einer Gebrauchseinheit zusammengefaßt sind.

## Claims

1. Agent for the detection of hydrogen peroxide or of hydrogen peroxide-forming substrates, consisting of 4-aminoantipyrine and an aromatic amine or phenol and a neutral to weakly acidic buffer, as well as possibly other additives and reagents known for the Trinder reaction, which form $H_2O_2$ with the substrate, characterised in that the 4-aminoantipyrine is, before use, present separately in a state unmixed by the first buffer and possibly the other reagents and is mixed with a small amount of a second buffer with a pH value of 8.5—14.0, whereby the two compositions are combined in a packaging unit or in a usage unit in an amount ratio to one another suitable for the detection.

2. Process for the detection of hydrogen peroxide or of hydrogen peroxide-forming substrates by means of 4-aminoantipyrine and an aromatic amine or phenol and a neutral to weakly acidic buffer, as well as possibly other additices or reagents known for the Trinder reaction which form $H_2O_2$ with the substrate, characterised in that, shortly before use, one combines 4-aminoantipyrine stabilised with a small amount of a second buffer with a pH value of 8.5—14.0 with an excess of the first buffer, as well as possibly the other reagents, adds the substrate thereto and evaluates the resultant coloration visually or photometrically.

3. Use of an agent consisting of two spatially separated compositions, one of which contains 4-aminoantipyrine and a small amount of a buffer with a pH value of 8.5—14.0 and the other an excess of a neutral or acidic buffer and both compositions contain further reagents necessary for the Trinder reaction or $H_2O_2$ formation for the detection of hydrogen peroxide or hydrogen peroxide-forming substrates, whereby the two compositions are combined in a packaging unit or in a usage unit in an amount ratio to one another suitable for the detection.

## Revendications

1. Agent pour la mise en évidence de peroxyde d'hydrogène ou de substrats formant du peroxyde d'hydrogène composé de 4-amino-antipyrine et d'une amine aromatique ou d'un phénol et d'un tampon de pH neutre à faiblement acide ainsi qu'éventuellement d'autres additifs et réactifs connus pour la réaction selon Trinder, formant de l'$H_2O_2$ avec le substrat, caractérisé en ce que la 4-amino-antipyrine se trouve sous une forme empêchant avant l'utilisation tout mélange avec le premier tampon et éventuellement avec les autres réactifs formant de l'$H_2O_2$ avec le substrat et est mélangée avec une faible cuantité d'un seqond tampon dont le pH est compris entre 8,5 et 14,0, les deux compositions étant réunies dans un rapport de quantités approprié à la réaction de mise en évidence dans une unité d'emballage ou dans une unité d'utilisation.

2. Procédé pour la mise en évidence de peroxyde d'hydrogène ou de substrats libérant du peroxyde d'hydrogène au moyen de 4-amino-antipyrine et d'une amine aromatique ou d'un phénol et d'un tampon de pH neutre à faiblement acide ainsi qu'éventuellement d'autres additifs et réactifs connus pour la réaction de Trinder formant de l'$H_2O_2$ avec le substrat, caractérisé en ce qu'on réunit de la 4-amino-antipyrine stabilisée par une faible quantité d'un second tampon dont le pH est compris entre 8,5 et 14,0, peu de temps avant l'utilisation avec un excès du premier tampon ainsi qu'éventuellement des autres réactifs, on ajoute le substrat et on évalue la coloration provoquée visuellement ou par photométrie.

3. Utilisation d'un agent comportant deux compositions séparée dans l'espace dont l'une comprend une 4-amino-antipyrine et une faible quantité d'un tampon dont le pH est compris entre 8,5 et 14,0 et l'autre un exès d'un tampon neutre ou acide et en ce que les deux compositions renferment d'autres réactifs nécessaires pour la réaction de Trinder ou la formation d'$H_2O_2$, pour la mise en évidence de peroxyde d'hydrogène ou de substrats formant du peroxyde d'hydrogène, les deux compositions étant combinées dans un rapport de quantités approprié à la mise en évidence, sous la forme d'une unité d'emballage ou d'une unité d'utilisation.